(19) Europäisches Patentamt
European Patent Office
Office européen des brevets

(11) Publication number: 0 186 102 B1

# (12) EUROPEAN PATENT SPECIFICATION

(45) Date of publication of the patent specification: **10.05.89**

(51) Int. Cl.⁴: **C07C 172/00**, C07C 49/513, C07F 7/18, A61K 31/59

(21) Application number: **85116099.4**

(22) Date of filing: **17.12.85**

(54) **Cholecalciferol derivatives.**

(30) Priority: **19.12.84 US 683442**

(43) Date of publication of application: **02.07.86 Bulletin 86/27**

(45) Publication of the grant of the patent: **10.05.89 Bulletin 89/19**

(84) Designated Contracting States: **AT BE CH DE FR GB IT LI LU NL SE**

(56) References cited:
**WO-A-83/00335**
**US-A- 4 248 791**

**CHEMICAL ABSTRACTS, vol. 96, no. 17, 26th April 1982, page 793, abstract 143178g, COLUMBUS, OHIO (US). TETRAHEDRON LETTERS, vol. 22, no. 43, 1981, pages 4309-4312, Pergamon Press Ltd. OXFORD (GB). YOSHIRO KOBAYASHI et al.: "Synthesis of 26,26,26-trifluoro-25-hydroxy and 27-nor-26,26,26-trifluoro-25-hydroxyvitamin D3"**
**THE JOURNAL OF THE AMERICAN CHEMICAL SOCIETY, vol. 104, no. 10, 19th May 1982, pages 2945-2948, WASHINGTON, DC (US). E.G. BAGGIOLINI et al.: Stereoselective total synthesis of 1alpha,25-dihydroxycholecalciferol"**

(73) Proprietor: **F. HOFFMANN-LA ROCHE & CO. Aktiengesellschaft, CH-4002 Basel(CH)**

(72) Inventor: **Baggiolini, Enrico, 30 Evergreen Drive, North Caldwell, N.J. 07006(US)**
Inventor: **Pizzolato, Giacomo, 194 Forest Avenue, Glen Ridge, N.J. 07028(US)**
Inventor: **Uskokovic, Milan, 253 Highland Avenue, Upper Montclair, N.J. 07043(US)**
Inventor: **Truitt, Gary, 109 Garner Avenue, Bloomfield, N.J. 07003(US)**

(74) Representative: **Lederer, Franz, Dr. et al, Van der Werth, Lederer & Riederer Patentanwälte Lucile-Grahn-Strasse 22, D-8000 München 80(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



ACTORUM AG

## Description

The invention relates to novel cholecalciferol derivatives, a process for their manufacture, novel intermediates therefor and medicaments based on the former cholecalciferol derivatives.

The cholecalciferol derivatives in accordance with the invention are the 26,26,26-trifluoro-1α,25-dihydroxycholecalciferol, particularly in form of the 25R-epimer essentially free of the 25S-epimer, and of the 25S-epimer essentially free of the 25R-epimer. In accordance with the invention, these compounds are prepared by reacting a compound of the formula

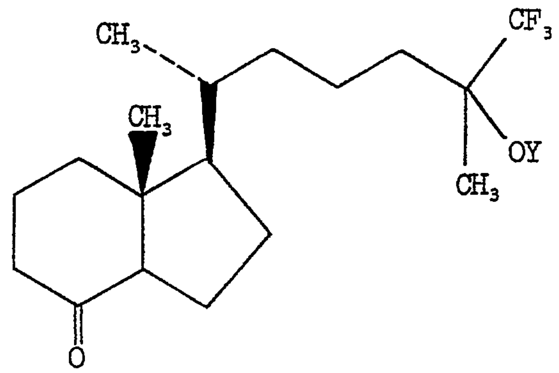

IV

where Y is trialkylated Si and, particularly where the hydroxylated C-atom has the R- or S-configuration, with the carbanion of [3S-(3α,5β,Z)]-2-[2-methylene-3,5-bis[(1,1-dimethylethyl)dimethylsilyloxy]cyclohexylidene]-ethyldiphenyl phosphine oxide and removing the silyl protecting groups.

The above reaction can be carried out at reduced temperatures, e.g. below –50°C, preferably at about –78°C, under an inert, e.g. an argan atmosphere, in an inert solvent, such as a cylic ether, preferably tetrahydrofurun (THF). The conversion of the phosphine oxide to the corresponding carbanion can be accomplished by initially treating the phosphine oxide with an alkyl lithium, e.g. b-butyl lithium.

Removal of the trialkylsilyl protecting group can be accomplished by treating the reaction product with a cation exchange resin under ambient conditions, in a solvent, such as a halogenated alkane, preferably methylene chloride. The resulting products can be purified e.g. by chromatography on silica gel.

As used throughout the specification and claims, "lower alkyl" refers to a straight or branched chain alkyl group with 1–8 C atoms, e.g. methyl, ethyl, n-propyl, i-propyl and t-butyl. "Aryl" means phenyl optionally substituted by alkyl, halogene, nitro, cyano and trifluoromethyl. Examples of aralkyl groups are benzyl and phenethyl.

The indenone starting materials utilized in the above mentioned process can be prepared as follows: [1R-[1β,(αS*, βS*],3aα,4aβ,7aβ]]-Octahydro-β,7a-dimethyl-4-[(1,1-dimethylethyl)dimethylsilyloxy]-α-ethenyl-1H-indene-1-ethanol is converted to [1R-[1β(R*),3aα,-4β,7aβ]]-1-(4-chloro-1-methyl-2-butenyl)octahydro-4-[(1,1-dimethylethyl)dimethylsilyloxy]-7a-methyl-1H-indene by treatment with thionyl chloride followed by pyridine. The resulting allylic chloride is then reacted with an aryl sulfinic acid salt, e.g. benzene sulfinic acid sodium salt, to yield the corresponding arylsulfonyl compound, e.g. [1R-[1β(R*),3aα,4β,7aβ]]-1-[(4-phenylsulfonyl)-1-methyl-2-butenyl]octahydro-4-[(1,1-dimethylethyl)dimethylsilyloxy] -7a-methyl-1H-indene. The aforesaid indene is then catalytically hydrogenated using a palladium-on-carbon (Pd/C) catalyst to provide the corresponding side chain saturated compound [1R-[1β(R*),3aα,4β,7aβ]]-1-[4-(phenylsulfonyl)-1-methylbutyl]-octahydro-4-[(1,1-dimethylethyl)dimethylsilyloxy]-7a-methyl-1H-indene. The completion of the side-chain to produce a compound of formula I below, is accomplished by reacting the carbanion of the aforesaid sulfonyl compound, formed by treatment with n-butyl lithium or lithium diisopropylamide, with trifluoroacetone.

In the obtained compound of the formula

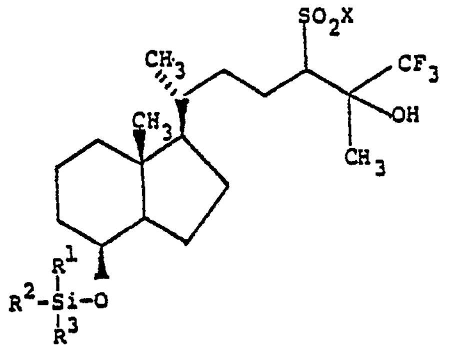

I

wherein X is aryl, and $R^1$, $R^2$ and $R^3$ are lower alkyl, aryl or aralkyl, preferably X is phenyl, $R^1$ and $R^3$ methyl, and $R^2$ t-butyl.

A compound of formula I, as a mixture of epimers at the 4 position in the side chain, can be dearysulfonylated with an alkali metal or with an alkali metal amalgam, in the presence of an alkali metal phosphate, in an inert organic solvent, such as a lower alkanol, a cyclic ether or preferably a mixture thereof. Preferred reactant are sodium amalgam and dipotassium hydrogen phosphate. Preferred solvents include methanol, THF and mixtures thereof. The alkali metal amalgam addition is carried out with cooling, preferably at temperatures below 0°C, most preferably at about –20°C.

The product of the reaction, after purification, e.g. by chromatography on silica gel, can be reacted with an epoxidation agent, such as a peracid, preferably trifluoroperacetic acid, at reduced temperatures, e.g. at 0°C. Preferably the reaction is carried out in the presence of an inorganic base, e.g. a phosphate base, preferably dipotassium hydrogen phosphate, in an inert organic solvent, e.g. a halogenated alkyl or aryl solvent, preferably methylene chloride. Under the conditions of the reaction, part of the resulting product may consist of the desilylated product. Thus the product of the reaction is a mixture of the protected and unprotected compounds of formula

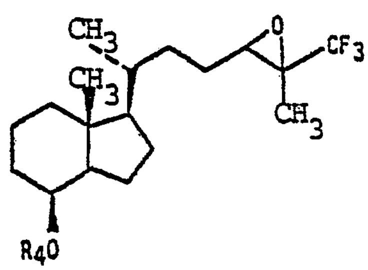

II

where $R^4$ is –Si($R^1$,$R^2$,$R^3$) or H, and $R^1$, $R^2$ and $R^3$ are as above.

After purification, e.g. flash by chromatography, one of the components of the above mixture or the two components of formula II can be treated with a reducing agent, e.g. an alkali metal or a metal hydride, preferably lithium aluminum hydride, in an inert organic solvent, e.g. an alkyl ether, such as ethyl ether, preferably under ambient conditions and under an inert atmosphere, e.g. of argon. There is thus produced the corresponding protected and/or unprotected compounds of formula

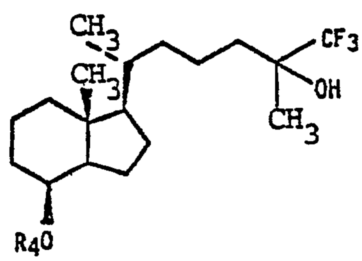

III

where $R^4$ is as above, as a mixture of epimers at the 5-position in the side chain. The compounds of formula III can be purified, e.g. by chromatography on silica gel. The compounds of formula III can then be treated with a cation exchange resin to deprotect any silylated compound and the mixture of epimers be separated by high performance liquid chromatography, after purification, e.g. by liquid chromatography on silica gel. To produce end products which are mixtures of epimers the high performance liquid chromatography can be eliminated.

The deprotected compound of formula III can then be treated with an oxidative agent, such as a chromate salt, particularly with a basic organic amine, e.g. a pyridinium halochromate, preferably pyridinium chlorochromate. The reaction is conveniently carried out under ambient conditions, in an inert solvent such as a halogenated alkane, e.g. a chloroalkane, preferably methylene chloride. There is obtained the ketone of the formula IV above, wherein Y stands for hydrogen.

The obtained ketone can then be treated without isolation, with a reagent introducing a trimethylsilyl protecting group on the side chain hydroxy, e.g. with trimethylsilylimidazole, at ambient temperature under an inert atmosphere. The resulting protected compounds have the formula IV, wherein Y is trialkylated Si.

The compounds of formula IV wherein Y is hydrogen or trialkylated Si, are novel and as such form part of the invention.

26,26,26-Trifluoro-1α,25-dihydroxycholecalciferol has vitamin $D_3$-like activity and exhibit anti-proliferative and differentiation-inducing effects. These anti-proliferative and differentiation-inducing effects on HL-60 cells can be demonstrated in vitro utilizing known procedures, e.g. as described in Progress in Cancer Research and Therapy, Vol. 23: Maturation Factors and Cancer, Ed. M.A.S. Moore, Raven Press, N.Y. 1982; Nature 270 (1977) 347–9 and Blood 54 (1979) 429–39. The results obtained are set out in the following table:

| Compound | Concentration[a] ($\times 10^{-9}$ molar) | Proliferation[b] HL-60 cells per ml $\times 10^{-4}$ | % reduction of cell number | Differentiation NBT reduction formazan "+" cells/total cells counted | %"+" |
|---|---|---|---|---|---|
| None (medium control) | | 84.7 ± 4.3 | – | 3/368 | <1 |
| Vehicle (0.1% ethanol) | | 78.9 ± 4.9 | 0 | 4/356 | 1 |
| 25R-compound | 1 | 59.1 ± 1.9 | 30 | 81/353 | 23 |
| 25R-compound | 10 | 27.2 ± 0.3 | 68 | 299/318 | 94 |
| 25R-compound | 100 | 22.9 ± 1.0 | 73 | 360/367 | 98 |
| 25R-compound | 1000 | 22.0 ± 0.7 | 74 | 340/344 | 99 |
| 25S-compound | 1 | 60.2 ± 3.9 | 29 | 84/334 | 25 |
| 25S-compound | 10 | 27.0 ± 1.4 | 68 | 338/354 | 96 |
| 25S-compound | 100 | 22.3 ± 2.6 | 74 | 323/330 | 98 |
| 25S-compound | 1000 | 20.0 ± 0.5 | 76 | 353/358 | 99 |

a Vehicle concentration in all experimental cultures was 0.1%, v/v, ethanol

b The cell viability in all cultures was greater than 97%. All cultures were initiated with $2 \times 10^4$ HL-60 cells per ml.

The data in the table indicate that the 26,26,26-trifluoro-1α,25-dihydroxycholecalciferol, particularly in the 25R- or 25S-form, restrain the proliferation of human promyelocytic tumor cells in vitro, even though they were not toxic to the cells. Furthermore, cells cultured in low concentrations of these compounds ($10^{-9}$ to $10^{-8}$ molar) were induced to differentiate toward a more mature cell type as evidenced by the acquisition of enzyme activity and cellular function. It can be expected that the above compounds are useful in treating diseases, such as neoplastic diseases, which owe in part to aberrant cellular proliferation and/or differentiation.

The above compounds can be administered in dosages in the range of about 0.10–3.0, preferably 0.25–2.0 µg/per day for the treatment of such disease states as osteoporosis, osteodystrophy, steroid induced osteopenia, hypoparathyroidism, hypophosphatemic rickets and hypophosphatemic osteomalacia, which are characterized by lower than normal levels of endogeneously produced 1α,25-dihydroxycholecalciferol. The above compounds can be administered in the same dosage range for the treatment of proliferative disease states, such as leukemia. They can be administered orally, subcutaneously, intramuscularly, intravenously, intraperitoneally or topically. They can be formulated into compositions, such as tablets, capsules or elixirs for oral administration, or in sterile solutions or suspensions for parenteral administration. About 0.10–3.0, preferably 0.25–2.0 µg of the above compounds can be compounded in a unit dosage, together with a pharmaceutically acceptable vehicle, carrier, preservative, stabilizer, binder, e.g. gum tragacanth, excipient, e.g. calcium phosphate, disintegrating agent, e.g. corn starch, lubricant, e.g. magnesium stearate, buffer, antioxidant, sweetening agent, e.g. sucrose, flavoring agent, e.g. peppermint. Various other materials may be present as coatings, e.g. shellac, or to otherwise modify the physical form of the dosage unit.

The above compounds can also be administered for the treatment of milk fever in pregnant ruminant animals prior to parturation in dosages in the range of 200–1000, preferably 25–200 µg/day using conventional formulations. For instance, sterile compositions for injection and/or topical administration can be formulated by dissolving or suspending the above compounds in a vehicle, e.g. a 10–20% ethanol(or propylene glycol)-water mixture, a naturally-occurring vegetable oil, e.g. sesame oil, or a synthetic fatty vehicle, e.g. ethyl oleate. They may also be formulated for intramuscular injection by suspension of 100–1500 µg thereof in a vehicle, e.g. a vegetable oil or a 80–95% ethanol (or propynene glycol)-water solution. They can also be formulated for oral administration by incorporation of 25–200 µg thereof into fatty acid pellets.

Example 1

a) A solution of 2.9 g (8.22 mmol) of [1R-[1β,[αS*,βS*],-3aα,4aβ,7aβ]]-octahydro-β,7a-dimethyl-4-[(1,1-dimethylethyl)dimethylsilyloxy]-α-ethenyl -1H-indene-1-ethanol in 100 ml of anhydrous ether was cooled at 0°C and treated dropwise and under argon with 2.76 ml (37.84 mmol) of thionyl chloride, followed by 0.276 ml of pyridine. The mixture was stirred at 0°C for 2 hours, then it was quenched by addition of 50 ml of a 2N sodium potassium tartrate solution. The ether phase was separated and the aqueous phase extracted with ethyl acetate. The combined organic phases were washed with 1N hydrochloric acid, water, 2N potassium bicarbonate and brine, dried and evaporated. The solvent was evaporated in vacuo and the residue was purified by chromatography on silica with hexane-ethyl acetate to give 2.9 g (95% yield) of pure [1R-[1β(R*),3aα,4β,7aβ]]-1-(4-chloro-1-methyl-2-butenyl)octahydro-4-[(1,1-dimethylethyl)dimethylsilyloxy] -7a-methyl-1H-indene as a low melting solid.

b) A solution of 2.9 g (7.81 mmol) of the allylic chloride of 1a) in 130 ml of hexamethylphosphoramide was treated with 10.1 g (61.52 mmol) of benzene sulfinic acid sodium salt and stirred at room temperature under argon for 24 hours. 130 ml of ice water were then added and, after stirring for 30 minutes, the mixture was extracted with ethyl acetate, the combined extracts were washed with water, dried and evaporated to dryness, and the residue was purified by chromatography through silica with hexane-ethyl acetate to give 3.5 g (94% yield) of pure [1R-[1β(R*),3aα,4β,7aβ]]-1-[(4-phenylsulfonyl)-1-methyl-2-butenyl]octahydro-4-[(1,1-dimethylethyl)dimethylsilyloxy] -7a-methyl-1H-indene, as a low melting solid.

c) A solution of 1.00 g (2.10 mmol) of the product of 1b) in 40 ml of ethanol was hydrogenated at room temperature and under normal pressure over 350 mg of 10% Pd/C. After 2 h, the catalyst was filtered and the filtrate was evaporated to dryness to give 1.00 g of [1R-[1β(R*),3aα,4β,7aβ]]-1-[4-(phenylsulfonyl)-1-methylbutyl]-octahydro-4 -[(1,1-dimethylethyl)dimethylsilyloxy]-7a-methyl-1H-indene.

d) A solution of 0.440 ml (3.14 mmol) of diisopropylamine in 10 ml of anhydrous THF was cooled at 0°C and treated dropwise under argon with 1.87 ml (2.99 mmol) of a 1.6 molar solution of n-butyllithium in hexane. After stirring for 15 minutes at 0°C, the resulting solution was cooled at –78°C and diluted with 10 ml of anhydrous THF. It was then treated dropwise with a solution of 1.00 g (2.09 mmol) of the sulfone of 1c) in 15 ml of THF and stirred at –78°C for 5 min, then at 0°C for 30 min. After cooling again at –78°C, the mixture was treated with 0.50 ml (5.59 mmol) of 1,1,1-trifluoroacetone and stirred at the same temperature for 1.5 h. It was then quenched by addition of 30 ml of a 1:1 mixture of 2N sodium potassium tartrate and 2N potassium bicarbonate, allowed to come to room temperature and extracted with methylene chloride. The organic extracts were washed with brine, dried and evaporated to dryness. The residue was purified by chromatography through silica with hexane-ethyl acetate, to give 0.71 g (72% yield) or [1R-[1β-(R*),3aα,4β,7aβ]]-octahydro-1-(6,6,6-trifluoro-5-hydroxy-1,5-dimethyl-4-phenylsulfonylhexyl)-4[(1,1-dimethylethyl)dimethylsilyloxy] -7a-methyl-1H-indene as a colorless oil.

e) A solution of 0.56 g (0.95 mmol) of the sulfone of 1d) as an epimeric mixture, in 18 ml of THF and 18 ml of methanol was treated with 10 g of dipotassium hydrogen phosphate and, after cooling at –20°C, with 11 g of 6% sodium amalgam. After stirring at –20°C for 10 min, 30 ml of brine were added. The mixture was allowed to come to room temperature and extracted with ethyl acetate. The organic extracts were washed with brine, dried and evaporated to dryness. The residue was purified by chromatography through silica gel with hexane-ethyl acetate to give 0.40 g of [1R-[1β(R*),-3aα,4β,7aβ]]-octahydro-1-[6,6,6-trifluoro-1,5-dimethyl-4-hexenyl]-4-[(1,1-dimethylethyl) dimethysilyloxy]-7a-methyl-1H-indene as a colorless oily mixture of geometrical isomers.

f) A solution of 0.260 ml (1.84 mmol) of trifluoroperacetic anhydride in 5 ml of anhydrous methylene chloride was cooled at 0°C, treated with 0.052 ml (1.89 mmol) of 90% hydrogen peroxide and stirred for 30 min. The resulting solution was added to 400 mg of the olefin of 1e) (0.92 mmol) and 1.5 g of dipotassium hydrogen phosphate in 5 ml of methylene chloride at 0°C. After addition, the mixture was allowed to come to room temperature for 30 min, quenched with 5 ml of a 10% aqueous solution of sodium sulfite, diluted with a 10% aqueous sodium bicarbonate solution and extracted with methylene chloride. The organic extracts were washed with brine, dried and evaporated to give 190 mg of pure protected product, [1R[1β(R*),3aα,4β,7aβ,]]-octahydro-1-(6,6,6-trifluoro-1,5-dimethyl-4,5-epoxyhexyl)-4-[(1,1-dimethylethyl) dimethylsilyloxy]-7a-methyl-1H-indene as a mixture of epimers, and 140 mg of desilylated product. After purification by flash chromatography, both products can be used in the next step.

g) To a suspension of 100 mg (2.63 mmol) of lithium aluminum hydride in 3 ml of anhydrous ether, a solution of 190 mg (0.42 mmol) of the protected product of 1f) in 2 ml of ether was added at room temperature

under argon atmosphere. The resulting mixture was stirred for 24 h, then quenced by addition of 2N aqueous sodium potassium tartrate and extracted with ether. The extracts were washed with brine, dried and evaporated to give, after purification over silica gel with hexane-ethyl acetate, 175 mg of pure thick oily [1R-[1β(R*),3aα,4β,7aβ]]-octahydro-1-(6,6,6,-trifluoro-5-hydroxy-1,5-dimethylhexyl)-4-[(1,1-dimethylethyl)dimethysilyloxy]-7a-methyl-1H-indene as a mixture of epimers.

h) A solution of 175 mg (0.39 mmol) of the mixture of epimers of 1g) in 9 ml of methanol was treated with 2.8 g of a cation exchange resin (AG 50W-X4, 200–400 mesh, Bio-Rad Laboratory, Richmond, CA) and stirred at room temperature for 12 days. The resin was removed by filtration and washed with methanol. The filtrates were evaporated to dryness. The residue was purified by filtration through silica gel with hexane-ethyl acetate. The two epimers were separated by liquid chromatography on silica gel (Magnum 9 Partisil-10 silica gel column, Whatman Inc., Clifton, NJ) with chloroform/ethyl acetate to give 65 mg of crystalline 5R* product [1R[1β(1R*,5R*),3aα,4β,7aβ]]-octahydro-1-(6,6,6-trifluoro -5-hydroxy-1,5-dimethylhexyl)-7a-methyl-1H-inden-4-ol, m.p. 110–111°C, $[\alpha]^{25}_{D}$ = +40.3° (c 0.2 in chloroform) and 55 mg of 5S* product [1R[1β(1R*,5S*), 3aα,4β,7aβ]]-octahydro-1-(6,6,6-trifluoro -5-hydroxy-1,5-dimethylhexyl)-7a-methyl-1H-inden-4-ol, $[\alpha]^{25}_{D}$ = +31.6° (c 0.2 in chloroform).

i) A solution of 50 mg (0.15 mmol) of the 5R* diol of 1h) in 2 ml of methylene chloride was added to a slurry of 97 mg (0.45 mmol) of pyridinium chlorochromate in 4 ml of methylene chloride. The resulting mixture was stirred at room temperature for 1.5 h, then diluted with 10 ml of ether, stirred for 15 min and filtered through kieselgur. The residue of the filtration was triturated with ether. The trituration extracts were combined, filtered and evaporated to dryness. The residue was purified by chromatography with hexan-ethyl acetate. The 49 mg of product were dissolved in 4 ml of anhydrous methylene chloride, treated with 0.13 ml (0.89 mmol) of trimethylsilylimidazole and stirred at room temperature under argon for 22 h. After addition of 0.5 ml of water, the mixture was stirred for 20 min, then diluted with water and extracted with ethyl acetate. The extracts were washed with water and brine, dried and evaporated to dryness. The residue was purified by chromatography with hexane-ethyl acetate, to give 51 mg of [1R-[1β(1R*,5R*),-3aα,4β,7aβ]]-octahydro-1-(6,6,6-trifluoro -5-trimethyl-silyloxy-1,5-dimethylhexyl)-7a-methyl-1H-inden-4-one, as a thick oil.

j) A solution of 141 mg (0.23 mmol) of [3S-(3α,5β,Z)]-2-[2-methylene-3,5-bis](1,1-dimethylethyl)-dimethylsilyloxy]-cyclohexylidene]ethyldiphenyl phosphine oxide in 6 ml of anhydrous THF was cooled at –78°C and treated dropwise under argon with 0.137 ml (0.22 mmol) of a 1.6 molar solution of n-butyllithium in hexane. After stirring for 5 min, a solution of 51 mg (0.13 mmol) of the ketone of 1i) in 1.5 ml of THF was added dropwise to the orange phosphinoxy carbanion solution.The resulting mixture was stirred at –78°C for 1.5 h, then treated with 3 ml of a 1:1 mixture of a 2N potassium sodium tartrate and a 2N potassium bicarbonate solution, allowed to come to room temperature, diluted with water and extracted with water and ethyl acetate. The combined organic layers were washed with brine, dried and evaporated to dryness. The residue was purified by filtration through silica gel, eluted with hexane-ethyl acetate, then dissolved in 0.5 ml of methylene chloride and 5 ml of methanol and stirred at room temperature over-night with 2 g of a cation exchange resin (AG 50W–X4). After filtration and evaporation of the solvent, the residue was purified by chromatography with hexane-ethyl acetate, to give 23 mg of 26,26,26-trifluoro-1α,25R-dihydroxycholecalciferol. $^1H$ NMR (400 MHz, $CD_3OD$) 0.60 (s, 3H), 0.99 (d, J=6.4, 3H), 1.30 (s, 3H), 4.15 (m, 1H), 4.38 (m, 1H), d 4.90 (br s, 1H), 5.31 (br s, 1H), 6.10 (dm, J=11.2 Hz, 1H), 6.34 (d, J=11.2 Hz, 1H).

<u>Example 2</u>

a) Following the procedure of Example 1i), 56 mg of 5S* diol of Example 1h) were converted to 59 mg of [1R-[1β(1R*,5S*),3aα,4β,7aβ]]-octahydro-1-(6,6,6,-trifluoro-5-trimethylsilyloxy-1,5-dimethylhexyl)-7a-methyl-1H-inden-4-one.

b) Following the procedure of Example 1j), 58 mg of the ketone of Example 2a) were converted to 59 mg of 26,26,26-trifluoro-1α,25S,dihydroxycholecalciferol, $^1H$ NMR (400 MHz, $CD_3OD$) 0.58 (s, 3H), 0.97 (d, J=6.8 Hz, 3H), 1.28 (s, 3H),d 4.13 (m, 1H),d 4.36 (m, 1H), 4.90 (br s, 1H), 5.29 (br s, 1H), 6.09 (d, J=11.2 Hz, 1H), 6.32 (d, J=11.2 Hz, 1H).

<u>Example A</u>

| Item | Ingredients | mg/capsule | | |
|---|---|---|---|---|
| 1. | Compound of the invention | 0.00010 | 0.00025 | 0.00050 |
| 2. | Polyethylene glycol 400 | 200.00 | 200.00 | 200.00 |
| 3. | Butylated hydroxy anisole | 0.100 | 0.100 | 0.100 |
| 4. | Ascorbyl palmitate | 1.00 | 1.00 | 1.00 |

Items 1, 3 and 4 were dissolved in item 2 under a blanket of nitrogen and encapsulated.

Example B

| Item | Ingredients | | |
|---|---|---|---|
| 1. | Compound of the invention | 0.10 mg | 0.50 mg |
| 2. | 95% ethanol–5% water | 2.00 ml | 3.00 ml |

Dissolve item 1 in item 2 under a blanket of nitrogen and inject intramuscularly.

## Claims

1. 26,26,26-Trifluoro-1α,25-dihydroxycholecalciferol.

2. The 26,26,26-trifluoro-1α,25R-dihydroxycholecalciferol essentially free of the 25S-epimer, and the 26,26,26-trifluoro-1α,25S-dihydroxycholecalciferol essentially free of the 25R-epimer.

3. A compound of the formula

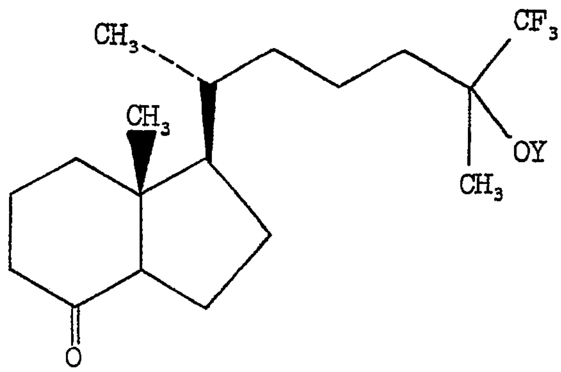

IV

where Y is H or trialkylated Si, particularly where the hydroxylated C-atom has the R- or S-configuration.

4. The compounds of claim 1 or 2 for use as therapeutically active substances, particularly in the treatment of milk fever in pregnant ruminants, in the treatment of disease states characterized by lower than normal levels of endogeneously produced 1α,25-dihydroxycholecalciferol, and as specific inducers of cell diferentiation and inhibitors of cell proliferation.

5. A process for producing the compounds of claim 1 or 2, which comprises reacting a compound of formula IV as defined in claim 3, wherein Y is trialkylated Si, with the carbanion of [3S-(3α,5β,Z)]-2-[2-methylene-3,5-bis[(1,1-dimethylethyl)dimethylsilyloxy]cyclohexylidene]ethyldiphenyl phosphine oxide and removing the silyl protecting groups.

6. A pharmaceutical composition on the basis of a compound according to claim 1 or 2.

7. The use of a compound according to claim 1 or 2 in the preparation of a pharmaceutical composition according to claim 6.

## Patentansprüche

1. 26,26,26-Trifluor-1α, 25-dihydroxycholecalciferol.

2. Das 26,26,26,Trifluor-1α, 25R-dihydroxycholecalciferol, im wesentlichen frei vom 25S-Epimeren, und das 26,26,26-Trifluor-1α, 25S-dihydroxycholecalciferol, im wesentlichen frei vom 25R-Epimeren.

3. Eine Verbindung der Formel

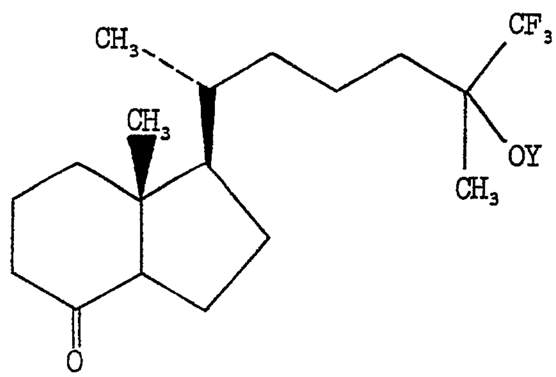

IV

worin Y Wasserstoff oder trialkyliertes Si, insbesondere worin das hydroxylierte C-Atom die R- oder S-Konfiguration hat.

4. Die Verbindungen der Ansprüche 1 oder 2 zur Verwendung als therapeutisch aktive Substanzen, insbesondere in der Behandlung des Milchfiebers bei tragenden Wiederkäuern, in der Behandlung von Krankheitszuständen, die charakterisiert sind durch unter dem Normalwert liegende Spiegel von endogen produziertem 1α,25-Dihydroxycholecalciferol, und als spezifische Induktoren der Zelldifferenzierung und als Inhibitoren der Zellproliferation.

5. Ein Verfahren zur Herstellung der Verbindungen von Anspruch 1 oder 2, welches darin besteht, daß man eine Verbindung der Formel IV, wie im Anspruch 3 definiert, worin Y trialkyliertes Si ist, mit dem Carbanion des [3S-(3α,5β,Z)]-2-[2-Methylen-3,5-bis[(1,1-dimethyläthyl)dimethylsilyloxy]cyclohexyliden]äthyldiphenyl-phosphinoxids reagieren lässt und die Silylschutzgruppen abspaltet.

6. Eine pharmazeutische Komposition auf der Basis einer Verbindung gemäß Anspruch 1 oder 2.

7. Die Verwendung einer Verbindung gemäß Anspruch 1 oder 2 bei der Herstellung einer pharmazeutischen Komposition gemäß Anspruch 6.

**Revendications**

1. Le 26,26,26-trifluoro-1α,25-dihydroxycholécalciférol.

2. Le 26,26,26-trifluoro-1α,25R-dihydroxycholécalciférol essentiellement exempt de l'épimère 25S et le 26,26,26-trifluoro-1α,25S-dihydroxycholécalciférol essentiellement exempt de l'épimère 25R.

3. Un composé de formule:

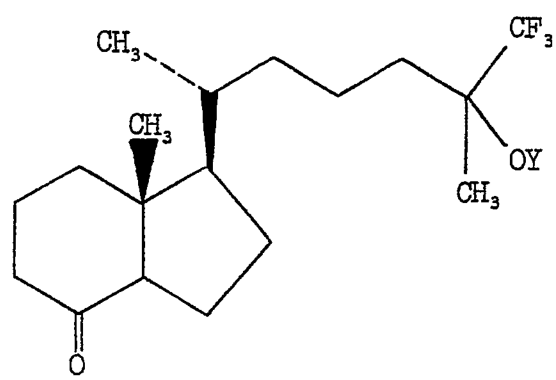

IV

dans laquelle Y représente H ou Si trialkylé, en particulier un tel composé dans lequel l'atome de C hydroxylé est en configuration R ou S.

4. Les composés de la revendication 1 ou 2, pour l'utilisation en tant que substances actives thérapeutiques, en particulier dans le traitement de la fièvre du lait chez les ruminants gravides, dans le traitement des états de maladie caractérisés par des niveaux inférieurs aux niveaux normaux de 1α,25-dihydrocholécalciférol de production endogène et en tant qu'inducteurs spécifiques de la différenciation cellulaire et inhibiteurs de la prolifération cellulaire.

5. Un procédé pour préparer les composés de la revendication 1 ou 2, qui consiste à faire réagir un composé de formule IV tel que défini dans la revendication 3, dans laquelle Y représente Si trialkylé, avec le carbanion de l'oxyde de [3S-(3α,5β,Z)]-2-[2-méthylène-3,5-bis-[1,1-diméthyléthyl)-diméthylsilyloxy]-cyclohexylidène]-éthyldiphénylphosphine puis à éliminer les groupes protecteurs silyle.

6. Une composition pharmaceutique à base d'un composé selon la revendication 1 ou 2.

7. L'utilisation d'u composé selon la revendication 1 ou 2 dans la préparation d'une composition pharmaceutique selon la revendication 6.